# EUROPÄISCHE PATENTANMELDUNG

(11) **EP 0 727 411 A2**
(43) Veröffentlichungstag der Anmeldung: **21.08.1996**
(21) Anmeldenummer: 96810076.8
(22) Anmeldetag: 06.02.1996
(51) Int. Cl.: C07C 213/02, C07C 217/92, C07C 41/22

(54) **Verfahren zur Herstellung von Diphenylaminen**

(30) Priorität: 14.02.1995 CH 432/95; 18.05.1995 CH 1475/95
(71) Anmelder: CIBA-GEIGY AG, 4002 Basel (CH)
(72) Erfinder: Brunner, Frédéric, Dr., CH-2054 Chézard (CH)

(57) **Zusammenfassung**

Beschrieben wird ein Verfahren zur Herstellung von Diphenylaminen der Formel 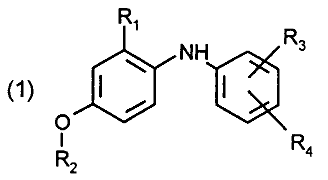
durch Oxybromierung einer Phenoletherverbindung mit elementarem Brom in Anwesenheit von Wasserstoffperoxid und einem Katalysator zu einer bromierten Phenoletherverbindung und anschliessender Reaktion dieser Verbindung mit einer Formanilidverbindung zur Verbindung der Formel (1).

Die Oxybromierungsreaktion stellt eine regioselektive, umweltfreundliche und kostengünstige Methode zur Herstellung von bromierten aromatischen Verbindungen dar.

Die Diphenylamine der Formel (1) stellen industriell wertvolle Zwischenprodukte für die Herstellung z.B. von Farbstoffen und insbesondere Farbbildnern vom Fluorantyp für druckoder wärmeempfindliche Aufzeichnungssysteme dar.

## Beschreibung

Die vorliegende Erfindung betrifft ein Verfahren zur Herstellung von Diphenylaminen der Formel 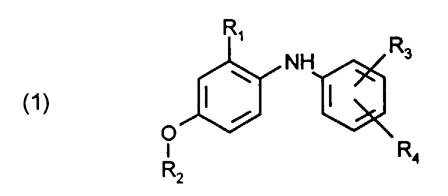 worin
R₁ und R₂, unabhängig voneinander, Niederalkyl; und
R₃ und R₄, unabhängig voneinander, Niederalkyl oder Halogen bedeuten.

Das Verfahren zur Herstellung von Diphenylaminen der Formel (1) durch Oxybromierung einer Phenoletherverbindung der Formel 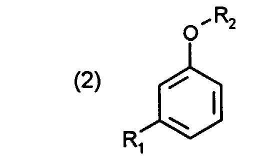 zur Verbindung der Formel

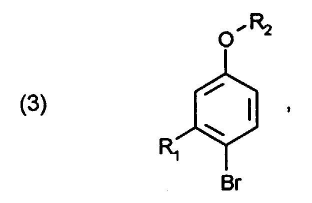

Umsetzung der bromierten Phenoletherverbindung der Formel (3) unter Ullmann-Kupplungsbedingungen mit einer Verbindung der Formel 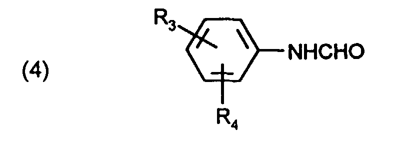 zur Verbindung der Formel (1) ist dadurch gekennzeichnet, dass man die Bromierung der Phenoletherverbindung der Formel (2) zur Verbindung der Formel (3) mit elementarem Brom und in Anwesenheit von Wasserstoffperoxid und gegebenenfalls eines Katalysators durchführt.

Der gesamte Reaktionsablauf lässt sich nach folgendem Schema darstellen:

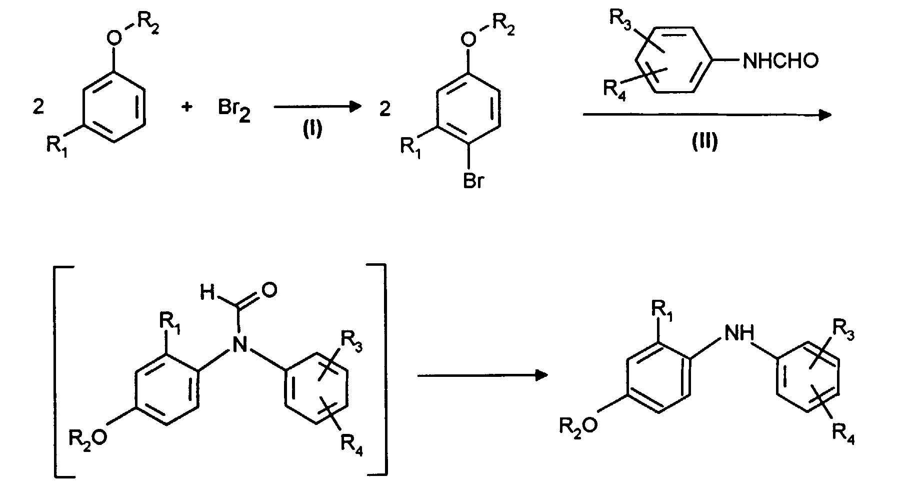

Niederalkyl entsprechend der Definitionen von R₁, R₂ und R₃ bedeuten Reste von geradkettigen oder verzweigten C₁-C₅-Alkanen, wie z.B. Methyl, Ethyl, Propyl, Isopropyl, Butyl, tert.Butyl oder Amyl. Vorzugsweise bedeuten die Reste R₁, R₂ und R₃ Ethyl und insbesondere Methyl.

Halogen bedeutet Fluor, Brom oder vorzugsweise Chlor.

Die Herstellung der Ausgangsverbindung der Formel (2) erfolgt in an sich bekannter Weise nach der Williamson-Synthese durch Erhitzen der entsprechenden Natriumphenolatverbindung mit einem Alkylhalogenid R₂-Hal in ethanolischer bzw. wässriger Lösung oder grosstechnisch durch Behandeln der alkalischen Phenollösung mit einem Dialkylsulfat (R₂)₂SO₂. Als Ausgangsverbindungen kommen z.B. m-Kresolethylether, m-Kresolpropylether oder vorzugsweise m-Kresolmethylether in Betracht.

Die Bromierung der Phenoletherverbindung (Reaktion (I)) verläuft z.B. vorzugsweise unter Verwendung von zwei Mol der Ausgangsverbindung der Formel (2), elementarem Brom, zwei Mol Wasserstoffperoxid und gegebenenfalls einem Katalysator, wie z.B. einem Ammoniumsalz und insbesondere Ammoniummolybdat. Weiterhin sind für das erfindungsgemässe Verfahren Katalysatoren geeignet, die z.B. in J.Chem.Soc.Chem.Comm. 19, 1421-1422 (1987) beschrieben sind.

In einer besonders bevorzugten Ausführungsform werden die Ausgangsverbindung, das Brom und Wasserstoffperoxid in stöchiometrischen Verhältnissen eingesetzt. Die Reaktion erfolgt dabei gewöhnlich so, dass man die Phenoletherverbindung der Formel (2), das Wasserstoffperoxid, das z.B. als 20 bis 70 gew.-%ige Lösung vorliegt und gegebenenfalls den Katalysator in Essigsäure (98%ig) vorlegt, stark rührt und das Reaktionsgemisch auf ca. 0°C abkühlt. Anschliessend lässt man das mit Essigsäure verdünnte Brom langsam zutropfen. Die Reaktion ist stark exotherm und wird bei einer Temperatur von z.B. -10 bis 80, vorzugsweise 0 bis 5°C durchgeführt. Die Reaktionszeit beträgt dabei z.B. 0,25 bis 10, vorzugsweise 0,5 bis 1 Stunden.

Die Aufarbeitung der entstandenen bromierten Phenoletherverbindung der Formel (3) erfolgt in an sich bekannter Weise durch Abtrennen und Einengen der halogenierten organischen Phase.

In dem erfindungsgemässen Oxybromierungsprozess (Reaktionsschritt (1) im oben dargestellten Reaktionsschema) wird für die Bromierung der Phenoletherverbindung der Formel (2) nur ein halbes Aequivalent Brom verbraucht. Die Reaktion (1) stellt daher eine regioselektive, umweltfreundliche und darüber hinaus auch kostengünstige Methode zur Herstellung von bromierten aromatischen Verbindungen dar.

In der Reaktion (11) reagiert die bromierte Phenoletherverbindung der Formel (3) mit dem Formanilid der Formel (4) in Anwesenheit von Kupfer und lod und Kaliumcarbonat unter CO₂-Abspaltung zum Diphenylamin der Formel (1). Die Reaktionstemperatur in diesem Reaktionsschritt beträgt z.B. 120 bis 250°C, die Reaktionszeit z.B. 1 bis 48, vorzugsweise 12 bis 25 Stunden. Im Unterschied zur Ullmann-Kupplung entsteht eine Mischung von Diphenylformamid und Diphenylamin. Das Gemisch der beiden Produkte wird anschliessend in wässriger Kaliumhydroxid-Lösung zum gewünschten Diphenylamin der Formel (1) hydrolysiert. Die Aufarbeitung des Diphenylamins geschieht in an sich bekannter Weise, beispielsweise durch Destillation, Kristallisation oder Extraktion.

Die Reaktion (11) ist allgemein bekannt und z.B. in Houben-Weyl, "Methoden der Organischen Chemie XII", Seite 32 bis 42 (1957) oder in "Organic Reactions III", Seite 19 bis 24 (1965) beschrieben.

Mit dem vorliegenden Verfahren lassen sich auf einfache Weise und in guten Ausbeuten Diphenylamine gewinnen, die industriell wertvolle Zwischenprodukte darstellen, wie z.B. für die Herstellung von Farbstoffen und vorzugsweise von Farbbildnem vom Fluorantyp für druck- oder wärmeempfindliche Aufzeichnungssysteme. Ausgehend von diesen Diphenylaminen und Hydroxybenzophenon-2'-carbonsäuren der Formel 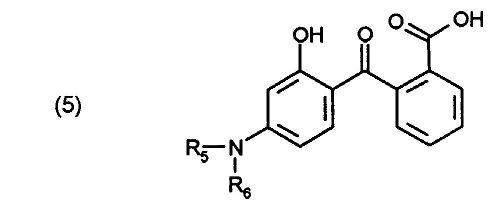 lassen sich beispielsweise Farbbildner der Formel (7) darstellen:

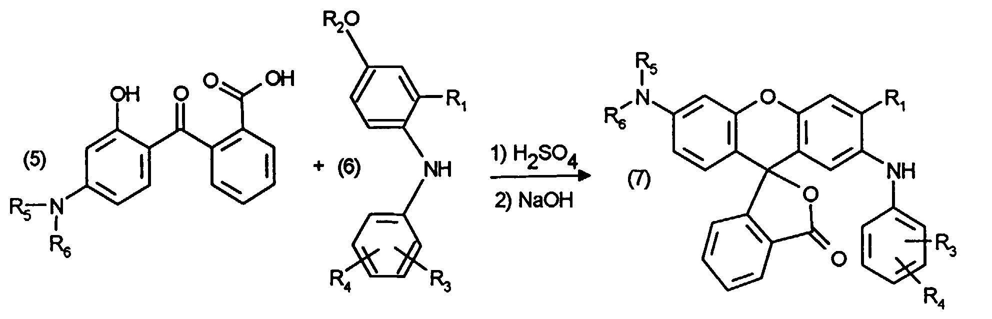

R₅ und R₆ bedeuten dabei, unabhängig voneinander, Niederalkyl. R₁, R₂, R₃ und R₄ haben die in den Formeln (1), (2), (3) und (4) angegebenen Bedeutungen.

Analog lassen sich auch Farbbildner der Formel 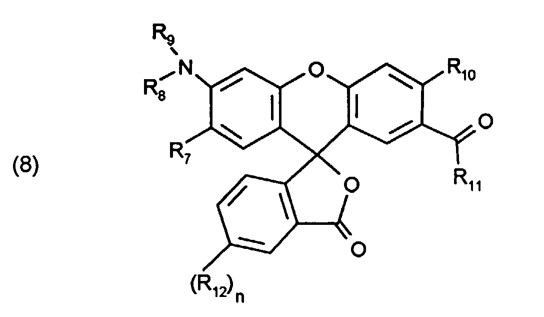 darstellen, worin
- R₇: Wasserstoff oder C₁-C₄-Alkyl;
- R₈ und R₉,: unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄-Alkyl; 2-Tetrahydrofuranyl, oder
- R₈ und R₉: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
- R₁₀: Wasserstoff, Hydroxy oder C₁-C₄-Alkyl;
- R₁₁: Wasserstoff; Hydroxy; C₁-C₈-Alkyl; C₁-C₈-Alkoxy; C₁-C₈-Halogenalkyl; unsubstituiertes oder durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
- R₁₂: Halogen; Nitro; C₁-C₄-Alkyl; C₁-C₄-Halogenalkyl; Amino; mono-C₁-C₄-Alkylamino; di-C₁-C₄-Alkylamino; oder COR₁₁; und
- n: 0; 1; 2; 3; oder 4;
bedeuten.

Farbbildner der Formel (7) bzw. druck- oder wärmeempfindliche Aufzeichnungssysteme sind z.B. aus der US-A-5,166,350, Farbbildner der Formel (8) bzw. druck- oder wärmeempfindliche Aufzeichnungssysteme aus der US-A-5,395,948 bekannt.

Farbbildner der Formel (8) lassen sich auch, wenn R₁₁ C₁-C₈-Alkyl bedeutet; durch Veresterung der Verbindung der Formel 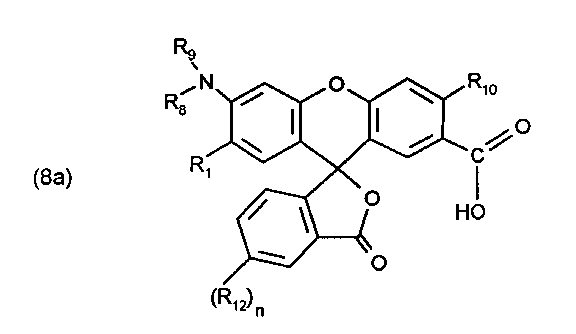 herstellen.

Als Veresterungsagentien können dabei Verbindungen verwendet werden, die eine C₁-C₈-Alkyl-, vorzugsweise Ethyl-Gruppe aufweisen, wie z.B. C₁-C₈-Alkylhalogenide, vozugsweise C₁-C₈-Alkylbromid, N'N-Dimethylformamid-di-C₁-C₈-alkylacetal, Tetraalkyloxonium-tetrafluoroborat oder Orthoameisensäure-tri-C₁-C₈-alkylester. Diese Herstellungsvariante der Farbbildner der Formel (8) bildet einen weiteren Erfindungsgegenstand.

Die folgenden Beispiele dienen der Veranschaulichung der Erfindung, ohne sie darauf zu beschränken.

### Beispiel 1: Herstellung von 2-Brom-5-methoxy-toluol

In einem mit einem Innenthermometer, Rührer und Tropftrichter ausgestatteten Planschliffkolben mit einem Volumen von 250ml werden 36,65g (0,3 mol) m-Kresol-Methylether und 39,8g (0,35 mol) 30%iges Wasserstoffperoxid in 40ml Essigsäure (98%ig) vorgelegt. Es wird stark gerührt und auf 0°C gekühlt. Anschliessend wird Brom, gelöst in 40ml 98%iger Essigsäure, langsam während 3 Stunden zugetropft. Die Reaktion ist stark exotherm. Die Reaktionstemperatur wird während der Zutropfzeit konstant auf ca. 0 bis 5°C gehalten. Nach Beendigung des Zutropfens lässt man die Temperatur auf 20°C ansteigen und rührt noch eine Stunde weiter. Danach stellt man den Rührer ab und lässt bei 20°C stehen. Es bilden sich sofort zwei Phasen. Die untere organische, halogenierte Phase wird von der oberen Essigsäure-Phase abgetrennt und eingeengt. Man erhält 2-Brom-5-methoxy-toluol der Formel 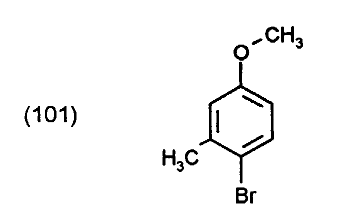 als gelbes Oel.

- Ausbeute (roh):: 58,12g (96,3 % d.Th.);
- GC-Analyse:: 98,5% Reinheit

### Beispiel 2: Herstellung von 4-Methoxy-2-methyl-diphenylamin

In einem mit einem Anker-Rührer, Thermometer, Kühler und Ölbad ausgestatteten 100ml-Sulfierkolben werden 12,1g (0,1 mol) Formanilid, 22,1g (0,11 mol) 2-Brom-5-methoxy-toluol der Formel (101), 13,8g (0,1 mol) wasserfreies Kaliumcarbonat, 2,0g (0,0079 mol) Jod und 1,6g (0,025 mol) Kupferpulver vorgelegt und gerührt. Man erwärmt auf 170°C und rührt 22 Stunden bei 170°C nach. Es entsteht eine schwarze Schmelze.

Man kühlt dann auf 80°C ab, gibt etwas Toluol hinzu und filtriert die Lösung ab. Der Rückstand wird mit Toluol nachgespült. Das Toluolfiltrat wird mit Spritzdampf abdestilliert (Destillat: Wasser/Toluol/unreagiertes 2-Brom-5-Methoxy-toluol). Als Rückstand bleibt ein Gemisch aus schwarzem Öl und Wasser. Das schwarze Öl wird mit 250g 50%iger Kaliumhydroxid-Lösung 25 Stunden unter Rückfluss gekocht. Das erhaltene Produkt wird in Toluol extrahiert und mit warmem Wasser gewaschen. Die Toluolphase wird mit Natriumsulfat getrocknet, abfiltriert und verdampft. Als Rückstand bleiben 17,0g der Verbindung der Formel 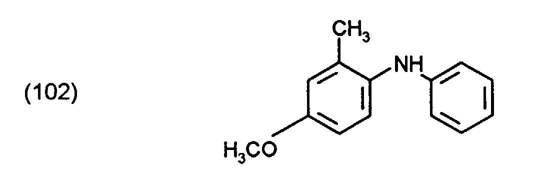 als schwarze Kristalle.
- Ausbeute:: 79,7 % d.Th.

### Beispiele 3 bis 8:

Man verfährt wie in den Beispielen 1 und 2 beschrieben. Man erhält die in der Tabelle 1 aufgeführten Verbindungen.

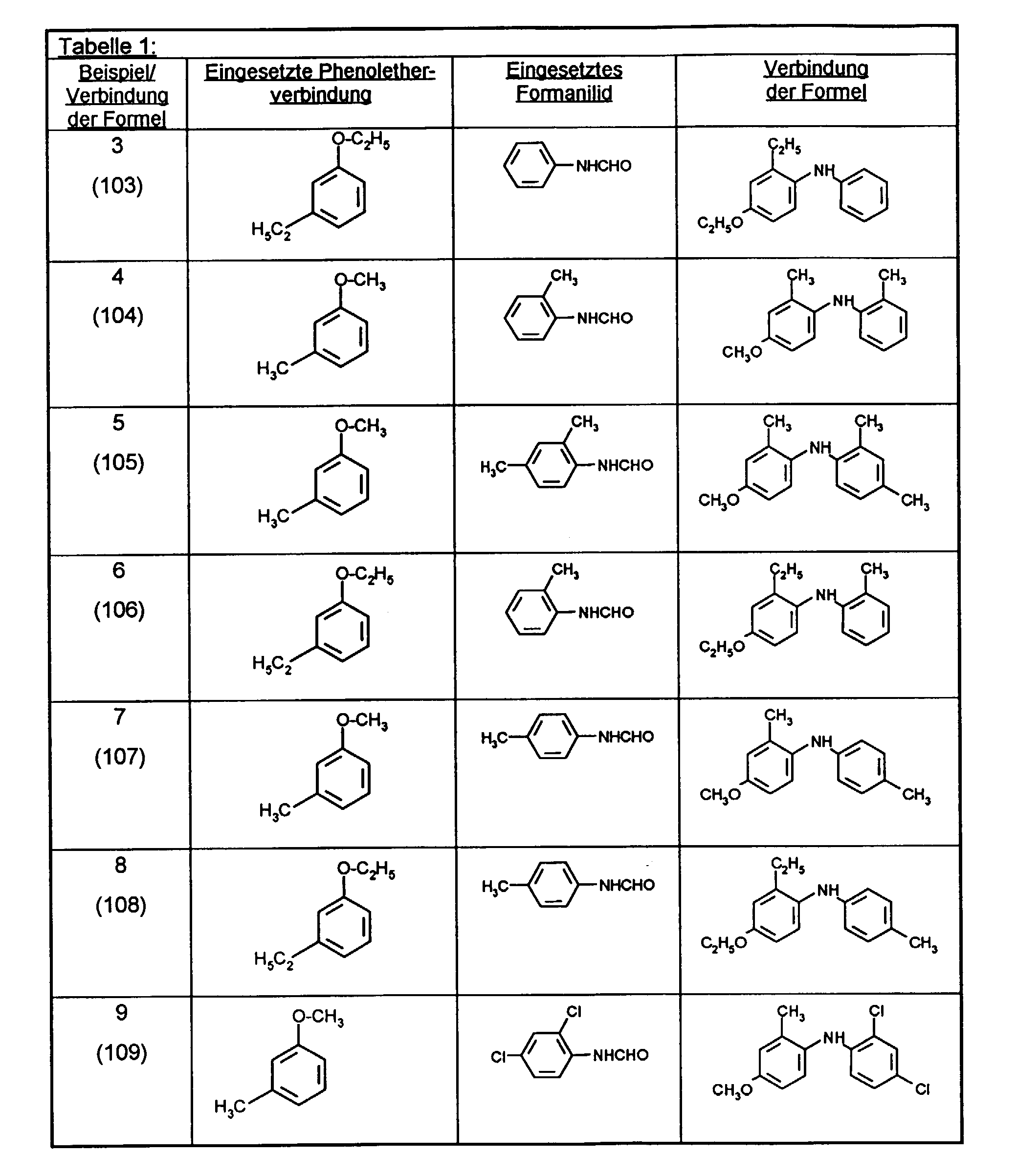

### Beispiel 10: Herstellung eines Fluoran-Farbbildners: Veresterung mit Ethylbromid:

29,5g des wasserfeuchten Produktes der Formel

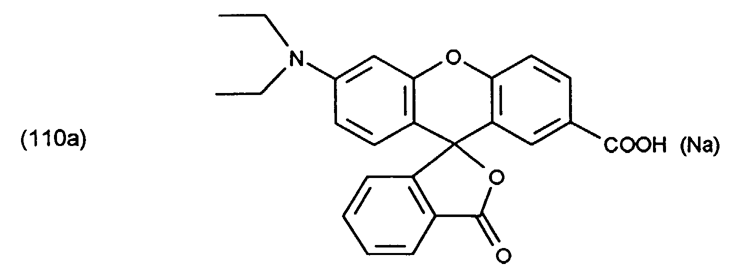

(Herstellung dieser Verbindung vgl. US-A-5,395,948)
werden in 46ml Wasser und 0,5g Tetrabutylammoniumbromid bei 25°C verrührt. Man gibt 0,1g Kaliumiodid zu und stellt den pH-Wert auf 10,4 mit 15,5ml 10N Natriumhydroxid-Lösung. Gleichzeitig erwärmt man auf 70-75 °C, wobei eine rote Lösung entsteht. Innerhalb von 8 Stunden dosiert man bei 70 bis 75°C gleichmässig 9,8g Ethylbromid zu und hält den pH-Wert durch Zugabe von weiteren 2,5ml 10N Natriumhydroxid-Lösung bei 8 bis 10. Man lässt noch während 15 Stunden bei 65 bis 70°C und konstantem pH-Wert nachrühren und filtriert das ausgefallene Produkt, wäscht mit einer Lösung von Natriumhydrogencarbonat, dann mit Wasser und schliesslich noch mit Methanol. Nach dem Trocknen erhält man 19g der kristallinen Verbindung der Formel 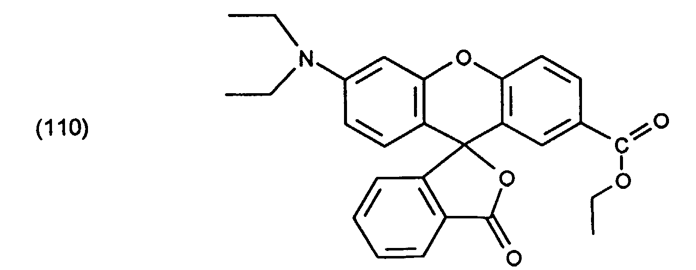
- Schmelzpunkt:: 151-153°C.

Das Produkt löst sich hervorragend in den üblichen Kapselölen und erzeugt als Farbbildner im Druck- sowie im Thermoverfahren ein intensives Orange.

### Beispiel 11: Herstellung eines Fluoran-Farbbildners: Veresterung mit N,N-Dimethylformamid-diethylacetal:

2g der trockenen Verbindung der Formel (110a) werden in 30ml Toluol bei 80°C gelöst. Dann gibt man 1,3g N,N-Dimethylformamid-diethylacetal zu und hält die Temperatur während 90 Minuten bei 90°C. Nun wird am Rotationsverdampfer eingeengt, der Rückstand in 23ml Isopropylalkohol heiss aufgenommen, kalt gerührt und der Niederschlag durch Filtration gewonnen. Nach dem Trocknen erhält man 1,5g der kristallinen Verbindung der Formel (110).
- Schmelzpunkt:: 150-151,5°C.

### Beispiel 12: Herstellung eines Fluoran-Farbbildners: Veresterung mit Orthoameisensäuretriethylester

4g der trockenen Verbindung der Formel (110a) werden in 26,7g Orthoameisensäuretriethylester eingerührt und auf 137°C erwärmt, wobei sich eine Lösung bildet. Man rührt 8 Stunden bei 137 bis 140°C und destilliert dann 20g des überschüssigen Orthoameisensäuretriethylesters ab. Der Destillationsrückstand (=5,6g) wird in 56ml Isopropylalkohol heiss aufgenommen, kalt gerührt und der Niederschlag durch Filtration gewonnen. Nach dem Trocknen erhält man 3g der kristallinen Verbindung der Formel (110). Schmelzpunkt: 147-149°C.

### Beispiel 13: Herstellung eines Fluoran-Farbbildners: Veresterung mit Triethyloxoniumtetrafluoroborat

4g der trockenen Verbindung der Formel (110a) werden in 75ml Methylenchlorid und 2ml N,N-Diisopropylethylamin bei 25°C verrührt. Bei 20-25°C werden in einer Stunde 4g Triethyloxoniumtetrafluoroborat eingetragen und während 15 Stunden nachgerührt. Nun werden die flüchtigen Anteile im Vakuum abdestilliert, der Rückstand in 50ml Isopropylalkohol heiss aufgenommen, klärfiltriert und unter Abkühlung kristallisiert. Nach Filtration und Trocknung erhält man 1,8g der Verbindung der Formel (110).
- Schmelzpunkt:: 149-150°C.

## Patentansprüche

1. Verfahren zur Herstellung von Diphenylaminen der Formel 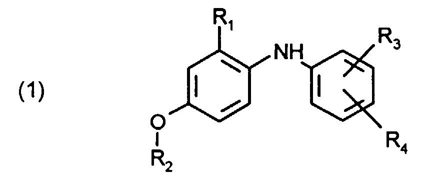 durch Oxybromierung einer Phenoletherverbindung der Formel 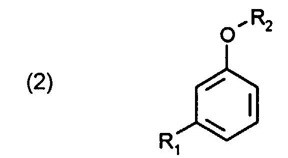 zur Verbindung der Formel 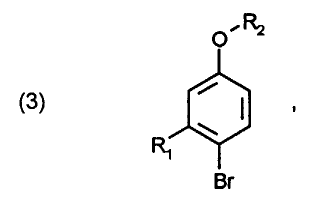 Umsetzung der bromierten Phenoletherverbindung der Formel (3) unter Ullmann-Kupplungsbedingungen mit einer Verbindung der Formel 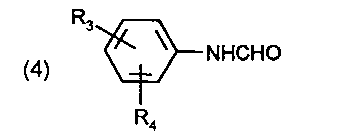 worin
R₁ und R₂, unabhängig voneinander, Niederalkyl; und
R₃ und R₄, unabhängig voneinander, Niederalkyl oder Halogen bedeuten, zur Verbindung der Formel (1), dadurch gekennzeichnet, dass man die Bromierung der Phenoletherverbindung der Formel (2) zur Verbindung der Formel (3) mit elementarem Brom und in Anwesenheit von Wasserstoffperoxid und gegebenenfalls eines Katalysators durchführt.

2. Verfahren nach Anspruch 1, dadurch gekennzeichnet, dass die Oxybromierung bei einer Temperatur von -10 bis 80°C durchgeführt wird.

3. Verfahren nach Anspruch 1 oder 2, dadurch gekennzeichnet, dass die Reaktionszeit für die Oxybromierung 0,25 bis 10 Stunden beträgt.

4. Verfahren nach einem der Ansprüche 1 bis 3, dadurch gekennzeichnet, dass bei der Oxybromierungsreaktion Wasserstoffperoxid, der Phenolether der Formel (2) und das Brom in stöchiometrischen Mengen eingesetzt werden.

5. Verfahren nach einem der Ansprüche 1 bis 4, dadurch gekennzeichnet, dass bei der Oxybromierung als Katalysator Ammoniummolybdat eingesetzt wird.

6. Verwendung der Phenyldiamine der Formel (1) als Zwischenprodukt für die Herstellung von Farbbildnem für druck- und wärmeempfindlichen Aufzeichnungssysteme.

7. Verfahren zur Herstellung von Farbbildnem der Formel 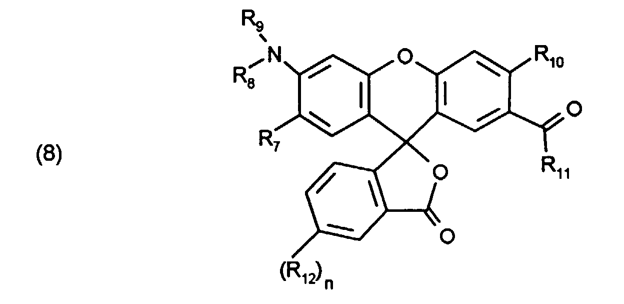 worin
R₇ Wasserstoff oder C₁-C₄-Alkyl;
R₈ und R₉, unabhängig voneinander, Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
R₈ und R₉ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₁₀ Wasserstoff, Hydroxy oder C₁-C₄-Alkyl;
R₁₁ C₁-C₈-Alkyl;
R₁₂ Halogen; Nitro; C₁-C₄-Alkyl; C₁-C₄-Halogenalkyla Amino; mono-C₁-C₄-Alkylamino; di-C₁-C₄-Alkylamino; oder COR₁₁; und
n 0; 1; 2; 3; oder 4;
bedeuten, durch Veresterung der Verbindung der Formel 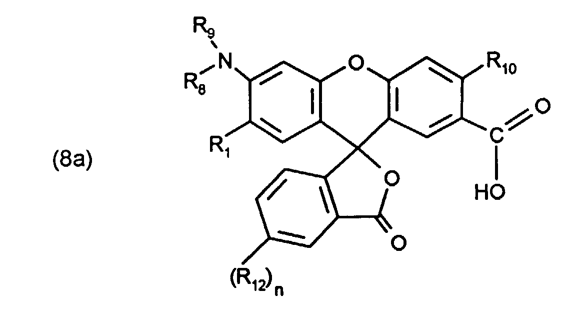 mit einer eine C₁-C₈-Alkylgruppe enthaltenden Verbindung.
